# EUROPEAN PATENT APPLICATION

(11) **EP 4 257 080 A1**
(43) Date of publication of application: **11.10.2023**
(21) Application number: 22382323.8
(22) Date of filing: 04.04.2022
(51) Int. Cl.: A61F 2/16, A61F 9/00, A61K 9/00

(54) **OCULAR BANDAGE**

(71) Applicant: Consejo Superior de Investigaciones Cientificas, 28006 Madrid (ES); Universidad de Valladolid, 47002 Valladolid (ES)
(72) Inventor: GUTIÉRREZ CONTRERAS, Rocío, 28006 Madrid (ES); FERNÁNDEZ, Mar, 28006 Madrid (ES); MARCOS CELESTINO, Susana, 28006 Madrid (ES); DE LA HOZ DURÁN, Andrés, 28006 Madrid (ES); MARTINEZ, Carmen, 47002 Valladolid (ES)
(74) Representative: Elion IP, S.L.

(57) **Abstract**

an ocular bandage (100, 100a), the ocular bandage (100, 100a) comprising:
- a membrane (10) comprising, at least, silk fibroin and polyethylene glycol; and,
- a central portion (20), the central portion blocking light within a pre-established wavelength range; and wherein
- the central portion (20) is dimensioned with respect to the membrane (10) such that there are at least outwardly located portions (30) of the membrane that are not covered by the central portion.

## Description

### TECHNICAL FIELD

The present disclosure generally relates to the field of ophthalmology.

### STATE OF THE ART

The cornea is the most anterior lens in the eye. It has two main functions: add refractive power to the eye and protect its internal structures. A transparent healthy cornea is crucial for proper vision. Corneal transparency and integrity can be compromised by abrasion, erosion or accidents involving chemical burns, which overall represent a 10% of the ophthalmological emergencies. A standard-of-care in the treatment of wounded corneas includes the use of eye drops and/or bandages. Eye drops can be easily administered by the patient, but they are partially lost with tear and need to be re-administered several times during the treatment. The use of corneal bandages increases and enhances the healing capacity of the cornea. Potential drugs and/or growth factors are retained by the bandage in contact with the cornea, enhancing its effect. There are two types of commercial corneal bandages: bandage contact lenses and amniotic membrane. Both have several drawbacks.

Bandage contact lenses are a long-time lasting method, since the contact lens needs to be placed onto the wounded cornea for an extended period of time to induce corneal healing. Due to the mechanical properties of the material and designs of the bandage contact lenses, the fact is that the bandage contact lens is not in total contact with the damaged cornea. Ultimately, contact lens wrapping could cause abrasive shear forces on the regenerating tissues. Oxygen permeability is also a limiting factor in bandage contact lenses; due to the fact that they have to be long-time worn, oxygen permeability has to be high. Another drawback of bandage contact lenses is that manipulation by the patient can pose risk of corneal infection.

Amniotic membranes also pose some issues. First, they are allogenic tissues, that is, the tissue is obtained from a human donor and may induce an immune response in the patient, resulting in rejection. Further, the amniotic membrane has to be sutured to the cornea. They need to be preserved at - 80°C. And finally, as a result of being donor dependent, they are scarce and expensive.

Several approaches have been explored in recent years with the aim of improving the current treatment options available for patient care. These approaches are framed in two main strategies: scaffold implantation, to promote cell migration and proliferation in the cornea, and membrane implantation, acting as a healing enhancer and external infection preventor.

Scaffold implantation is based on the development of biocompatible materials that enhance fibroblasts and epithelial cells migration and proliferation in the healing process.

In situ-formed collagen/polyethylene glycol hydrogels have been developed with good healing results. These hydrogels are formed directly on the damaged cornea.

OcuPair^{™} is an adhesive hydrogel for temporary corneal repair (up to 72 hours) and wound stabilization. It is composed of a biocompatible photo-crosslinkable polyamidoamine dendrimer and biodegradable photo-crosslinkable hyaluronic acid. It is designed to be directly applied on the corneal wound, where it is irradiated with blue light to initiate crosslinking.

These hydrogels presumably work by forming a scaffold which will be populated with collagen fibers. This is a major challenge, as cornea transparency requires high degree of collagen organization, and reproducing the natural pattern of collagen packing, orientation and interweaving that that of a natural healthy cornea appears to be challenging.

Membrane implantation consists in obtaining ex-situ membranes to be placed on top of the cornea, generally secured to the corneal surface during a surgical procedure. These artificial membranes would replace the use of amniotic membranes, which as previously stated, are the current standard-of-care.

There are several approaches currently under development related to membrane implantation.

One approach is cyclodextrin crosslinked collagen implants. These are non-degradable preshaped implants, requiring corneal suturing, aiming at supporting re-epithelialization. As the implant is non-degradable, nor removable, fine-tuning its shape and refractive index to prevent refractive alterations is most critical.

Other membrane approaches are based on collagen-PEG, but they need to be sutured to the cornea. BIOcular^{™} dressing overcomes this issue, since it is attached to the cornea through a suture-less light-initiated mechanism. It is made of a nanofiber-reinforced chitosan membrane, which is photobonded to the ocular surface. While the suture-less approach and degradability would overcome drawbacks of previous approaches, the apparent lack of biocompatibility and the complexity of the manufacturing process appears to have made it non-viable. Further, up to date, no *in vivo* experiments have been carried out.

### DESCRIPTION OF THE INVENTION

The present disclosure intends to solve the shortcomings of prior-art solutions by providing an ocular bandage based on silk fibroin for the treatment of corneal abrasions.

A first aspect of the invention relates to an ocular bandage, the ocular bandage comprising:
- a membrane comprising, at least, silk fibroin and polyethylene glycol; and,
- a central portion, the central portion blocking light within a pre-established wavelength range;
the central portion being dimensioned with respect to the membrane such that there are at least outwardly located portions of the membrane that are not covered by the central portion.

These outwardly located portions of the membrane not covered by the central portion are configured to be adhered to ocular tissue, for instance, by means of photobonding.

The proposed ocular bandage overcomes the drawbacks of the existing solutions, since:
- Silk fibroin is an inert material, with low immunogenicity, i.e. does not induce an immune response, which is an expected property for any biomaterial. Further, silk fibroin is naturally produced, so it is abundant and affordable compared to amniotic membrane.
- It provides a suture-less solution, since the silk fibroin membrane can be attached to the cornea through photobonding.
- It does not require expensive storage equipment.

In certain embodiments the membrane comprises around 3% silk fibroin and around 5% polyethylene glycol of total volume of a solution used to cast the membrane. This specific proportion allows for faster healing of the wound where the ocular bandage is applied, while at the same time providing sufficient rigidity to the membrane.

In certain embodiments the outwardly located portions of the membrane comprise a photo-initiating agent; for instance, those outwardly located portions can be coated with a photo-initiating agent. The photo-initiating agent is activatable by light, the light having the pre-established wavelength range. The photo-initiating agent can be is a solution containing riboflavin or Rose Bengal.

In certain embodiments, the central portion is made of a hydrophobic material. This way, when the photo-initiating agent is delivered to the outwardly located portions (for instance, by means of a syringe or by drops), the central portion remains unstained.

In certain embodiments the central portion is made of a flexible material and/or semirigid material. Making the central portion of a flexible material facilitates the adaptation of ocular bandage to the ocular tissue. Making the central portion of a semirigid material -usually having increased rigidity with respect to the membrane- facilitates removing the central portion after the ocular bandage has been put in place and bonded onto the ocular tissue.

In certain embodiments the ocular bandage further comprises a peripheric portion, which is also arranged for blocking light within a pre-established wavelength range. This further peripheric portion may be ring-shaped and having suitable dimensions for covering -and thus protecting- the limbus. This peripheric portion can partially cover the membrane or it can be dimensioned as a separate element, not overlapping with the membrane.

In certain embodiments the membrane further comprises a growth factor for stimulating the growth of the wounded tissues and improving healing times. It is also possible that the membrane comprises a therapeutic drug, for preventing, treating or relieving symptoms of a disease or abnormal condition.

The ocular bandage as disclosed may have a thickness of between 50 µm and 150 µm. A membrane having thickness below 50 µm can be difficult when handling it. Keeping the membrane thickness below 150 µm provides the advantage that the membrane is not wrinkled -especially around its periphery- when implanted into the cornea; and the implanted membrane is not uncomfortable for the end user.

The central portion can be embodied as a sticker, which is adhered to the membrane. This way, the central portion can be easily removable after staining and/or after implantation. The adherence of the sticker is preferably in lower that the adherence of the bonds produced after the ocular bandage is implanted on the ocular tissue, to facilitate removal of the central portion. It is also possible that a non-sticky surface of the central portion is provided with some gripping means, for enabling removal of the central portion, if the adherence is too strong.

In some embodiments the central portion fully covers at least a pupil of the eye.

In some embodiments the at least outwardly located portions of the membrane that are not covered by the central portion fully surround the central portion. If the ocular bandage is made circular, the outwardly located portions of the membrane are a single peripheral ring or annular portion around the central portion.

An ocular bandage as disclosed in the foregoing can be used in the treatment of corneal damage.

Another aspect of the disclosure relates to a kit for implanting an ocular bandage onto ocular tissue; the kit comprises
- an ocular bandage;
- a photoinitiating agent for at least partially impregnating the corneal bandage; and,
- a light source for providing light of a wavelength tuned to the excitation wavelength of the photo-initiating agent.

In certain embodiments, the ocular bandage is as defined in the foregoing in respect with the first aspect.

In certain embodiment of the kit, the photoinitiating agent is a solution containing riboflavin or Rose Bengal and the light source provides light having UV, blue and/or green wavelength.

A further aspect of the disclosure relates to a method for obtaining an ocular bandage; the method comprising:
i. stirring a silk fibroin solution with polyethylene glycol;
ii. pouring the resulting stirred solution into a flat mold;
iii. shaking the mold to ensure homogeneity distribution.

A further aspect of the invention relates to a method for implanting an ocular bandage on an eye, the method comprising:
- staining a periphery of an ocular bandage with photoinitiating agent; the ocular bandage is according to the first aspect as defined in the foregoing;
- placing the ocular bandage on the eye, with the central portion upwards with respect to the surface of the eye;
- irradiating at least at least outwardly located portions of the ocular bandage with light, such that the ocular bandage is photochemically bonded to the ocular tissue of the eye;
- removing the central portion of the ocular bandage.

In certain embodiments the method further comprises soaking the ocular bandage in a growth factor solution.

In certain embodiments light is irradiated during less than 400 s. And the step of irradiating with light can be carried out at an irradiance below 0.075 W/cm².

In certain embodiments the photoinitiating agent solution concentration is below 0.01% (w/v).

The different aspects and embodiments of the invention defined in the foregoing can be combined with one another, as long as they are compatible with each other.

Additional advantages and features of the invention will become apparent from the detailed description that follows and will be particularly pointed out in the appended claims.

### BRIEF DESCRIPTION OF THE DRAWINGS

To complete the description and in order to provide for a better understanding of the invention, a set of drawings is provided. Said drawings form an integral part of the description and illustrate embodiments of the invention, which should not be interpreted as restricting the scope of the invention, but just as examples of how the invention can be carried out. The drawings comprise the following figures:
Figure 1 shows a possible embodiment of a corneal bandage according to the present disclosure.
Figure 2 shows another possible embodiment of a corneal bandage.
Figure 3 shows InfraRed spectra of silk fibroin and silk fibroin-PEG membranes.
Figure 4. Stretching forces after irradiation with 0.075W/cm² and 10⁻² % RB
Figure 5. Stretching forces after irradiation with 0.075W/cm² and 10⁻³ % RB
Figure 6. Stretching forces after irradiation with 0.019W/cm² and 10⁻⁴ % RB

### DESCRIPTION OF WAYS OF CARRYING OUT THE INVENTION

The following description is not to be taken in a limiting sense but is given solely for the purpose of describing the broad principles of the invention. Embodiments of the apparatus and method of the present disclosure will be described by way of example, with reference to the accompanying drawings.

Silk fibroin-based bandages have been developed, aimed at overcoming the drawbacks found in the previous technologies. In the last years silk fibroin-based biomaterials have been developed with applications ranging from biomedicine (tissue engineering, implants, drug delivery) to food industry. Silk fibroin is extracted from silkworms' silk cocoons. The raw silk contains fibroin and sericin. After extraction and purification, silk fibroin can be conformed into membranes, scaffolds, drops... with high biocompatible, non-cytotoxic and low immunogenic properties. In our formulation, polyethylene glycol (PEG) is added to the silk fibroin, adding porosity to increase oxygen permeability in the material. This membrane approach is suture-less, and the silk fibroin bandage herein disclosed can be attached to the cornea through a photobonding method, allowing full total contact with the cornea. Moreover, it is a ductile material, which adapts its shape to the corneal curvature, reducing potential abrasive shear forces. The degradation rate of the membranes can be adjusted through chemical procedures in the manufacturing process. The control of the degradation rate allows designing membranes which dissolve within hours or days to membranes with a high-residency time, which act as removable corneal bandages. Growth factors or drugs can be administered with the bandage to improve wound healing. Also, the versatility of the photobonding process allows partial bonding of the membrane (for example a peripheral ring, which could create a central reservoir), and control of the bonding force to favor peeling off the membrane. These silk fibroin membranes are affordable, compared to amniotic membrane and other manufactured corneal membranes, and do not need expensive storage equipment.

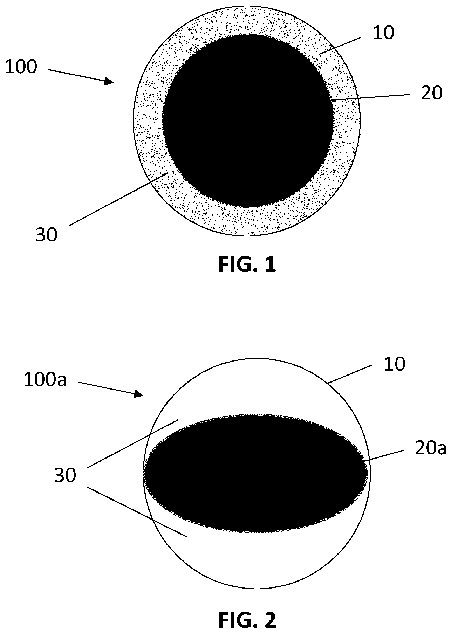

Figure 1 shows a possible embodiment of a corneal bandage 100 according to the disclosure. The corneal bandage 100 comprises a silk fibroin membrane 10 and a mask 20 which in the embodiment shown is a circular black mask, for blocking light. In this embodiment the portion of the membrane not covered by the black mask is a peripheral ring 30.

Figure 2 shows another possible embodiment of a corneal bandage 100a. In this case the corneal bandage 100a comprises a silk fibroin membrane 10 and a mask 20a which in the embodiment shown is black, for blocking light, but it is oval. In this embodiment there are two portions 30 of the membrane not covered by the black mask.

The bandages are made of silk fibroin solution and polyethylene glycol. Both solutions are stirred together and poured in a flat mold at room temperature for water to evaporate.

The resulting ocular bandage can be peripherally photobonded to the injured cornea. This allows for healing of the bound, as demonstrated with in a corneal wound healing model in a fibroblast cell culture: healing of the wound happened after 15 days in the presence of the silk fibroin bandage. The control samples did not have external adjuvants and there was no self-healing of the cornea.

The bandage can be produced in different forms leading to different administration and removal strategies:
- In a possible embodiment, the ocular bandage can be sealed and integrated into the cornea and reabsorbed;
- It is also possible that the bandage is removed after the cornea is healed.

In the present approach, the silk fibroin ocular bandage is photobonded to the cornea, through the use of Rose Bengal (photosensitizer) and green light irradiation. Rose Bengal and green light parameters have been adjusted to obtain an optimal membrane photobonding to the cornea with the minimum photosensitizer concentration and light irradiance

The biocompatibility of the herein proposed ocular bandages has been demonstrated with corneal fibroblasts cultures in *vitro.*

### Experimental

Silk fibroin (SF) was extracted from Bombyx mori silk cocoons. Cocoons were initially cut in small pieces and degummed to remove sericin in boiling 0.2 M Na₂CO₃ solution for 40 min. After degumming, fibroin fibers were repeatedly rinsed in distilled water and allowed to dry at 60°C overnight. Afterwards, the dry fibers were dissolved in 9.3 M LiBr aqueous solution for 4 hours at 60° C. The final solution was dialyzed against water for 48 hours at 4° C using dialysis membranes (MWCO 3.5 kDa, Spectrum). Finally, the resulting solution was centrifuged to remove impurities. The final concentration of silk fibroin was 7-8% in water solution.

In order to prepare the bandages (SF-PEG), 3% silk fibroin solution was stirred with 5% polyethylene glycol (300 MW) for 2 minutes. The resulting solution was poured in a flat mold at controlled temperature and humidity conditions for water to evaporate. To ensure homogeneity distribution in the membrane, a shaker was used. PEG is a porogen and produces pores in the membrane. In addition, PEG induces β-sheets, crosslinking the fibers and preventing the membrane from dissolving in water.

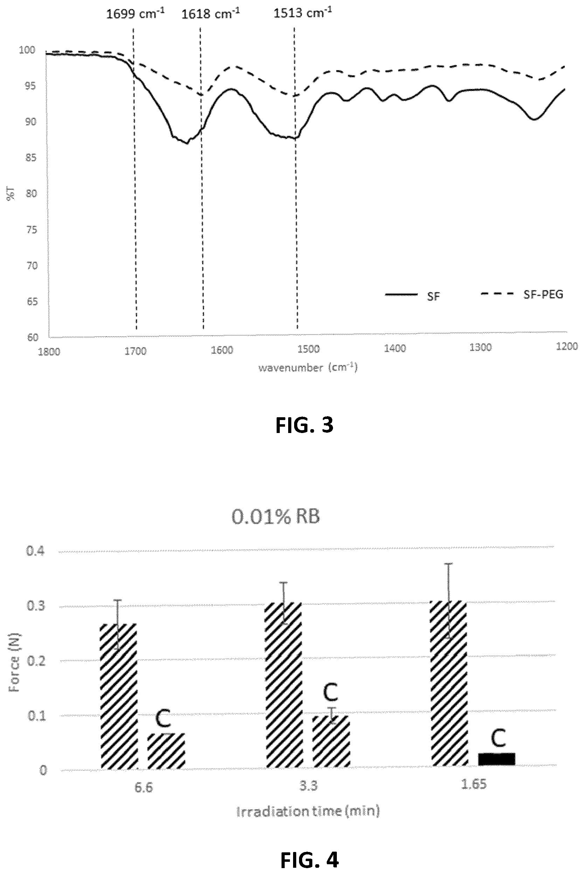

The presence of β-sheets is characterized with IR spectroscopy. Figure 3 shows the IR spectra of SF-PEG membranes and control membranes (SF), fabricated without PEG. The three marked bands are characteristic of β-sheets.

The effect of degumming and cross-linking on the stability and degradation of the membranes was studied. In particular, SF-PEG membranes prepared with silk fibroin extracted for 30 and 40 minutes were analyzed. In addition, the effect of cross-linking procedure (cross-linker and soaking time) on the SF-PEG membranes was studied: 75% methanol for 15 minutes; 70% ethanol for 20 minutes; and 70% isopropanol for 20 minutes.

The degradation rate of the SF-PEG membranes was assessed in PBS, lysozyme (LS) 500 U/mL and protease XIV (PT) 0.05 U/mL.

Degumming times did not affect membrane stability. Based on this and on the results from in vitro experiments, the preparation of all our SF-PEG membranes with silk fibroin degummed for 40 minutes was established. Increased crosslinking time confers the membranes a greater stability in PBS and lysozyme. All membranes degrade within the first 24 hours in protease.

With the aim of optimizing the concentration and irradiation parameters, rabbit corneal strips were photobonded to membrane SF-PEG strips at different RB concentrations and green light irradiations. 15x3 mm rabbit corneal strips were photobonded to 15x3 mm SF-PEG membrane strips. Characterization of the bonding forces was performed using uniaxial stretching. They were measured after irradiation with different irradiances and RB concentrations. The stretching forces of RB-stained samples and their controls (non-RB-stained), both irradiated, vary with irradiation time (as shown in Figures 4-6). Samples stained with RB and irradiated showed higher stretching forces than their controls (C). Due to the flexibility of the SF-PEG membranes, samples with higher irradiance deformed before bond detaching or membrane breaking, as seen in the dashed bars.

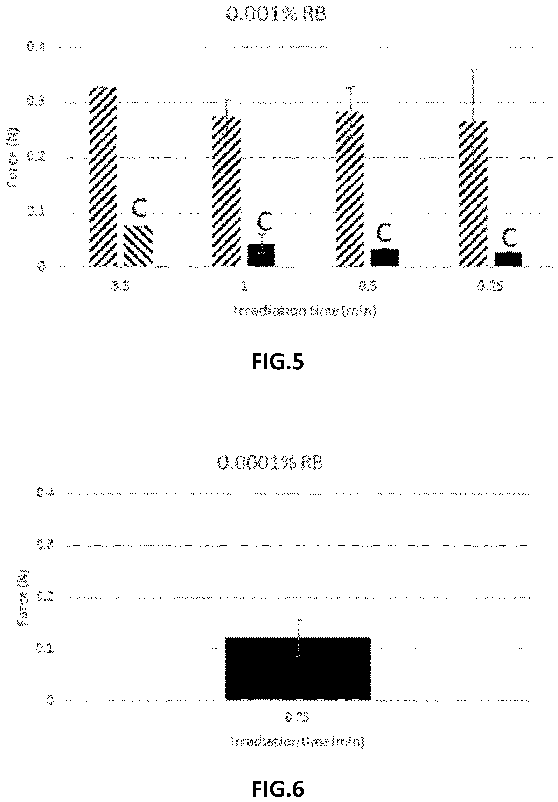

Bonding forces were studied at different irradiances (0.019 - 0.075 W/cm²), irradiance times (0.25 - 6.6 minutes) and RB concentrations (10⁻¹ - 10⁻⁴ %). Irradiance and RB concentration parameters were decreased until reaching a threshold in which cornea and membrane strips detached before deformation of the membrane. These parameters are 0.019W/cm² and 10⁻⁴ % RB (Figure 5). The control samples at these parameters could not be measured because the bonding was negligible and the sample could not be mounted in the stretcher instrument.

As part of the study carried out, the response of corneal stroma cells to silk fibroin-derived membranes (SFMs) following injury was evaluated.

A stromal in vitro wound model was used to evaluate the effect of SFMs on the different processes occurring during stromal repair. Briefly, transparent SFMs were placed on the bottom of Petri dishes, on top of which human corneal stromal cells (HCSCs) were seeded and cultured. When HCSCs reached confluence a linear wound was made. Similar cultures without SFMs were used as control group. Wound closure time and cell organisation were evaluated by microscopy. Proliferation and myofibroblast differentiation were analysed by immunocytochemistry. Hepatocyte growth factor (HGF) secretion was measured by ELISA.

The result is that silk fibroin-derived membranes induced a significant faster wound closure than control showing complete closure at day 3. HCSCs showed higher organisation on the substrate during the wound closure than in control. A peak of proliferation was observed at day 1 in both groups but it was significantly increased in HCSCs seeded on SFMs (p<0.001). On day 3, the percentage of proliferation decreased in both groups showing no differences. HGF secretion was significantly increased by SFMs at day 3 compared to control (p<0.05). No myofibroblast differentiation was observed in HCSCs seeded on SFMs at any study time compared to a low percentage of myofibroblasts observed in control at day 3.

The study showed the positive effects of SFMs during the stromal wound closure. The SFMs could improve the corneal repair process and prevent the development of corneal opacities in view of the promotion of faster wound closure. The faster closure appears to be caused by an earlier proliferation peak in presence of SFMs, but may also indicate SFMs support of the migration process. The absence of myofibroblasts with SFMs may result from a higher HGF secretion, which is has been shown to inhibit myofibroblast generation in the corneal stroma.

## Claims

1. An ocular bandage (100, 100a), the ocular bandage (100, 100a) comprising:
- a membrane (10) comprising, at least, silk fibroin and polyethylene glycol; and,
- a central portion (20), the central portion blocking light within a pre-established wavelength range; and wherein
- the central portion (20) is dimensioned with respect to the membrane (10) such that there are at least outwardly located portions (30) of the membrane that are not covered by the central portion.

2. The ocular bandage (100, 100a) of claim 1, wherein the membrane comprises around 3% silk fibroin and around 5% polyethylene glycol of total volume of a solution used to cast the membrane (100, 100a).

3. The ocular bandage (100, 100a) of any previous claim, wherein the outwardly located portions of the membrane comprise a photo-initiating agent activatable by light, the light having the pre-established wavelength range.

4. The ocular bandage (100, 100a) of any previous claim, wherein the central portion is made of a hydrophobic material.

5. The ocular bandage (100, 100a) of any previous claim, wherein the central portion is made of a flexible material and/or of a semirigid material.

6. The ocular bandage of any previous claims, further comprising a peripheric portion (30), the peripheric portion blocking light within a pre-established wavelength range.

7. The ocular bandage (100, 100a) of any previous claim, wherein the membrane has a thickness of between 50 µm and 150 µm.

8. The ocular bandage (100, 100a) of any previous claim, wherein the membrane (10) and the central portion (20) are adhered to each other.

9. The ocular bandage (100, 100a) of any previous claim for use in the treatment of corneal damage.

10. A kit for implanting an ocular bandage (100, 100a) onto ocular tissue, the kit comprising:
- an ocular bandage (100, 100a);
- a photoinitiating agent for at least partially impregnating the corneal bandage;
- a light source for providing light of a wavelength tuned to the excitation wavelength of the photo-initiating agent.

11. A kit according to claim 10, wherein the ocular bandage is according to any of claims 1-9.

12. A method for implanting an ocular bandage on an eye, the method comprising:
i) staining a periphery of an ocular bandage with photoinitiating agent; the ocular bondage being according to any of claims 1-9;
ii) placing the ocular bandage on the eye, with the central portion upwards with respect to the surface of the eye;
iii) irradiating at least the outwardly located portions of the ocular bandage with light;
iv) removing the central portion from the ocular bandage.

13. The method of claim 12, which further comprises soaking the ocular bandage in a growth factor solution.

14. The method of any of claims 12-13, in which the step of irradiating with light is carried out at an irradiance below 0.075 W/cm² and/or in which light is irradiated during less than 400 s.

15. The method of any of claims 12-14, in which the photoinitiating agent solution concentration is below 0.01% (w/v).
